# FASCICULE DE BREVET EUROPEEN

(11) **EP 3 275 423 B1**
(45) Date de publication et mention de la délivrance du brevet: **05.02.2020**
(21) Numéro de dépôt: 17183477.3
(22) Date de dépôt: 27.07.2017
(51) Int. Cl.: A61K 8/31, A61K 8/39, A61K 8/49, A61K 8/60, A61Q 19/00, A61K 8/81, A61K 8/06

(54) **COMPOSITION COSMÉTIQUE SOUS FORME D'ÉMULSION HUILE DANS EAU PRÉPARABLE À TEMPÉRATURE AMBIANTE**
KOSMETISCHE ZUSAMMENSETZUNG IN FORM EINER ÖL-IN-WASSER-EMULSION, DIE BEI ZIMMERTEMPERATUR HERGESTELLT WERDEN KANN
COSMETIC COMPOSITION IN THE FORM OF AN OIL-IN-WATER EMULSION WHICH CAN BE PREPARED AT ROOM TEMPERATURE

(30) Priorité: 27.07.2016 FR 1657233
(43) Date de publication de la demande: 31.01.2018
(73) Titulaire: BTL Cosmetics, 75015 Paris (FR)
(72) Inventeur: BATTAIL, Lucile, 75015 PARIS (FR)
(74) Mandataire: Agasse, Stéphane

(56) Documents cités:
- EP-A1- 3 015 100
- WO-A1-2016/056589
- US-A1- 2010 022 665

## Description

La présente invention concerne un procédé de préparation d' une composition cosmétique sous forme d'une émulsion huile dans eau qui est réalisable à température ambiante.

Dans le cadre de la présente invention, on entend par composition cosmétique réalisable à température ambiante, une composition cosmétique qui est obtenue sans la nécessité de mettre en œuvre une étape de chauffage. Par température ambiante, il s'agit d'une température qui est comprise entre environ 15°C et 30°C, généralement d'environ 20°C.

Les compositions cosmétiques réalisables à température ambiante sont particulièrement prisées dans l'industrie cosmétique car :
- du fait de l'absence d'étapes de chauffage, leur préparation est rapide ; ce qui procure un gain de temps ;
- elles permettent d'effectuer des économies d'énergie ;
- elles préservent la valeur active des principes actifs sensibles à la chaleur que peuvent contenir de telles compositions.

En outre, les compositions cosmétiques réalisables à température ambiante présentent l'intérêt qu'elles sont parfaitement appropriées pour être fabriquées dans le cadre d'un usage domestique par leur utilisateur final, et le cas échéant de manière personnalisée. En effet, pour des raisons de sécurité, ainsi que de facilité de préparation, il est particulièrement avantageux de fournir à des utilisateurs des compositions cosmétiques réalisables à la maison, par exemple sous forme d'un kit, qui ne nécessitent pas d'étapes de chauffage et/ou d'agitation mécanique requérant un appareillage particulier qui n'est souvent pas à leur portée. La mise en œuvre de compositions cosmétiques à la maison connaît un fort engouement dans le domaine des produits cosmétiques. En particulier, on cherche à proposer aux consommateurs des compositions cosmétiques qui soient préparables à la maison et de manière personnalisable, c'est-à-dire en tenant compte de leurs souhaits spécifiques sur les propriétés recherchées des compositions cosmétiques qu'ils vont fabriquer.

Pour toutes ces raisons, on est incité à mettre au point de nouvelles compositions cosmétiques qui soient réalisables à température ambiante.

Les compositions cosmétiques se présentent généralement sous la forme d'émulsions. Lorsqu'on travaille à température ambiante, il est très difficile d'épaissir et d'enrichir les émulsions, car on ne peut pas utiliser des composés solides à température ambiante qui nécessitent d'être fondus par chauffage tels que des hydrocarbures de haut poids moléculaire (vaselines, paraffines), des acides gras de haut poids moléculaire, des alcools gras de haut poids moléculaire, des esters gras de haut poids moléculaire, des cires végétales, animales ou encore minérales.

C'est pourquoi, il est aujourd'hui particulièrement difficile de formuler des émulsions très compactes et très crémeuses qui soient réalisables à température ambiante.

La présente invention se propose de surmonter les inconvénients qui ont été détaillés ci-dessus en proposant un nouveau procédé de préparation d'une composition cosmétique sous forme d'une émulsion huile dans eau qui est réalisable à température ambiante et qui présente des propriétés de compacité et d'onctuosité qui sont particulièrement recherchées dans le domaine des cosmétiques.

L'inventeur de la présente invention a en effet mis au point de manière tout à fait surprenante une composition cosmétique qui remplit parfaitement ces objectifs et présente d'autres avantages qui seront détaillés ci-dessous.

Le procédé selon la présente invention conduit ainsi à une composition cosmétique sous forme d'une émulsion huile dans eau qui comprend au moins :
- un latex inverse ;
- un tensio-actif hydrophile qui est choisi parmi les esters de polyglycérol qui sont liquides à température ambiante et de HLB supérieur ou égal à 8 ;
- une phase aqueuse ;
- une phase grasse,
le rapport du pourcentage massique dudit tensio-actif sur le pourcentage massique dudit latex inverse étant compris entre 0,1 et 1, plus préférentiellement compris entre 0,15 et 0,6, lesdits pourcentages massiques étant exprimés par rapport à la masse totale de ladite composition cosmétique.

HLB est l'acronyme anglophone de « Hydrophilic-Lipophilic Balance » qui se traduit par « équilibre hydrophile/lipophile ».

Dans le cadre de la présente invention, on entend par « latex inverse » (connu également sous la dénomination anglophone de « inverse polymer emulsion »), une émulsion eau dans huile dont la phase aqueuse dispersée contient au moins un polymère replié qui se déploie lorsqu'une phase aqueuse est ajoutée à ladite émulsion de telle sorte que par un mécanisme d'inversion de phases on obtienne une émulsion huile dans eau dont la phase continue est un gel aqueux se présentant sous la forme de microsphères de gel.

Ces latex inverses comprennent avantageusement au moins un tensio-actif, généralement dénommé « tensio-actif inverseur », qui favorise l'inversion de phases au moment de l'ajout de la phase aqueuse.

De tels latex inverses sont parfaitement connus de l'homme du métier pour leur utilisation en tant qu'agents épaississants ou émulsionnants dans des compositions cosmétiques, notamment pour des gels-crèmes.

Les demandes de brevet FR 2 774 996 A1, FR 2 808 446 A1, EP 1828 256 A2, EP 1 233 750 A1, EP 1444 973 A1, et EP 2 646 510 A1 décrivent des latex inverses qui sont parfaitement appropriés pour la mise en œuvre des compositions cosmétiques préparées selon l'invention.

Dans un mode de réalisation de l'invention, le latex inverse peut être une composition comprenant une phase huile, une phase aqueuse, au moins un agent émulsionnant de type eau dans huile, au moins un agent émulsionnant de type huile dans eau, comprenant de 20% à 70% en masse, et de préférence de 25% à 50% en masse, d'un polyélectrolyte branché ou réticulé, ledit polyélectrolyte est :
- soit un homopolymère à base d'un monomère possédant ou bien une fonction acide fort partiellement ou totalement salifiée, ou bien une fonction acide faible partiellement ou totalement salifiée,
- soit un copolymère à base d'au moins un monomère possédant une fonction acide fort, copolymérisé ou bien avec au moins un monomère possédant une fonction acide faible ou bien avec au moins un monomère neutre,
- soit un copolymère à base d'au moins un monomère possédant une fonction acide faible, copolymérisé avec au moins soit un monomère neutre, soit un monomère possédant une fonction acide faible.

Par exemple, le latex inverse peut être choisi parmi les produits de la société SEPPIC commercialisés sous les dénominations commerciales suivantes :
- SEPIGEL® 305 (nom INCI : Polyacrylamide, C13-C14 isoparaffine et Laureth-7),
- SEPIGEL® 501 (nom INCI : C13-14 isoparaffine, huile minérale, polyacrylate/polyacrylamide de sodium et polysorbate 85),
- SEPIGEL® 502 (nom INCI : C13-C14 isoparaffine, isostéaryl isostéarate, polyacrylate de sodium, polyacrylamide et polysorbate 60),
- SIMULGEL® EG (nom INCI : acrylate de sodium, copolymère de acryloyldimethyl taurate, isohexadecane et polysorbate 80),
- SIMULGEL® NS (nom INCI : hydroxyethyl acrylate, copolymère de acryloyldimethyl taurate, squalane and polysorbate 60),
- SIMULGEL® A (nom INCI : polyacrylate d'ammonium, isohexadecane, polysorbate 89),
- SIMULGEL® 600 (nom INCI : acrylamide, copolymère de acryloyldimethyl taurate, polysorbate 80),
- SIMULGEL® 800 (nom INCI : polyacryloyldimethyl taurate de sodium, isohexadecane et sorbitan oléate),
- SIMULGEL® HT (nom INCI : polyacrylamide, huile de paraffine, polysorbate 80),
- SIMULGEL® INS100 (nom INCI : hydroxyéthyl acrylate, copolymère d'acryloyldiméthyl taurate de sodium, isohexadecane et polysorbate 60),
- SIMULGEL® SMS88 (nom INCI : polymère croisé d'acrylate-acryloyldimethyltaurate-diméthylacrylamide de sodium, isohexadecane et polysorbate 60),
- SIMULGEL® EPG (nom INCI : acrylate de sodium, copolymère d'acryloyldimethyl taurate, polyisobutène, caprylyl capryl glucoside),
INCI étant l'acronyme anglophone pour "International Nomenclature of Cosmetic Ingredients" qui se traduit par « nomenclature internationale des ingrédients cosmétiques ».

Dans un mode de réalisation de l'invention, le latex inverse est choisi parmi ceux dont le tensio-actif inverseur est non éthoxylé. Cela présente l'avantage que le latex inverse qui est un des constituants essentiels de la composition cosmétique est d'origine naturelle. Par exemple, il peut s'agir du SIMULGEL® EPG.

De manière avantageuse, le latex est choisi dans le groupe constitué par le SIMULGEL® EPG, le SIMULGEL® INS100 et le SIMULGEL® EG.

De manière tout à fait préférée, le latex inverse est le SIMULGEL® EPG.

De manière avantageuse, le HLB du tensio-actif est compris entre 8 et 14, plus préférentiellement entre 9 et 13,5, et encore plus préférentiellement entre 10 et 13.

Ledit tensio-actif est choisi parmi les esters de polyglycérol.

Les esters de polyglycérol sont constitués d'une partie hydrophobe (acide gras) et d'une partie hydrophile (glycérol(s)). En plus de leurs propriétés tensioactives, ces esters présentent des propriétés émollientes et hydratantes qui sont particulièrement bénéfiques pour la composition cosmétique préparée selon l'invention.

De préférence, le tensio-actif est un ester de polyglycérol qui comprend 3 à 10 glycérols, plus préférentiellement 5 à 6 glycérols, et 1 à 3 acides gras saturés ou insaturés. Les acides gras sont avantageusement des acides gras saturés, plus préférentiellement choisis parmi l'acide laurique, myristique, palmitique, stéarique, caprique, caprylique, isostearique et oléique.

L'ester de polyglycérol peut être choisi dans le groupe constitué par polyglycéryl-2 caprate, polyglycéryl-2 laurate, polyglycéryl-3 laurate, polyglycéryl-4 laurate, polyglycéryl-4 isostéarate, polyglycéryl-4 oléate, polyglycéryl-5 laurate, polyglycéryl-5 oléate, polyglycéryl-5 dioléate, polyglycéryl-6 caprylate, polyglycéryl-6 tricaprylate, polyglycéryl-10 laurate, polyglycéryl-10 myristate, polyglycéryl-10 isostéarate, polyglycéryl-10 diisostéarate, polyglycéryl-10 oléate et polyglycéryl-6-isostéarate.

De manière tout à fait avantageuse, le tensio-actif est le polyglycéryl-6 isostéarate.

Dans un mode de réalisation de l'invention, la composition cosmétique sous forme d'une émulsion huile dans eau comprend au moins :
- un SIMULGEL® EPG ;
- un polyglycéryl-6 isostéarate ;
- une phase aqueuse ;
- une phase grasse.

De manière tout à fait surprenante, l'inventeur a découvert qu'un latex inverse en combinaison avec un tensio-actif choisi parmi ceux décrits ci-dessus permettait d'obtenir à température ambiante des compositions cosmétiques présentant de meilleures propriétés de stabilité, de compacité et/ou d'onctuosité que celles des compositions cosmétiques également obtenues à température ambiante et connues à ce jour.

Par ailleurs, la composition cosmétique présente des propriétés de compacité et d'onctuosité qui égalent, voire même qui surpassent celles des compositions cosmétiques mettant en œuvre des corps gras tels que des hydrocarbures de haut poids moléculaire, des acides gras de haut poids moléculaire, des alcools gras de haut poids moléculaire, des esters gras de haut poids moléculaire, des cires végétales, animales ou encore minérales et qui requièrent donc des étapes de chauffage et d'agitation mécanique vigoureuse au cours de leur préparation afin de fondre lesdits corps gras.

L'inventeur a en effet découvert un effet synergique entre les latex inverses et les tensio-actifs hydrophiles choisis parmi les esters de polyglycérol liquides à température ambiante et de HLB supérieur ou égal à 8, qui est fort avantageux pour obtenir à température ambiante, et de surcroît rapidement et sans nécessiter d'agitation vigoureuse, des compositions cosmétiques qui demeurent stables au cours du temps et aux propriétés de texture pouvant être également très variées afin de correspondre aux multiples attentes de leurs consommateurs.

Les compositions cosmétiques préparées selon l'invention sont perçues au toucher comme étant très agréables. Elles ne présentent pas de sensation collante.

Les compositions cosmétiques préparées selon l'invention pénètrent rapidement dans la peau et procurent une sensation de douceur à son utilisateur lors de leur application.

De plus, les compositions cosmétiques préparées selon l'invention présentent l'avantage de rester parfaitement stables au cours du temps. En effet, leur stabilité a été testée et validée à l'étuve à une température de 45°C. On a observé que les compositions cosmétiques préparées selon l'invention demeuraient parfaitement stables, et ce après plus de six mois de stockage.

A partir de cette association tout à fait innovante consistant en un latex inverse et un tensio-actif choisi parmi ceux décrits ci-dessus, la composition cosmétique préparée selon l'invention présente l'avantage que selon les quantités du latex inverse et du tensio-actif mises en œuvre et selon le ratio des quantités de ces constituants essentiels de la composition, ladite composition selon l'invention peut présenter des textures très variées : allant d'une forme liquide légère (par exemple un sérum) à une forme riche et compacte (par exemple une crème épaisse et riche), et ce avec des constitutions de phases aqueuse et grasse inchangées.

Dans une composition cosmétique sous forme d'émulsion huile dans eau, un tensio-actif parmi ceux décrits ci-dessus à lui seul permettrait uniquement d'obtenir des textures du type lotion ou lait liquide. En outre, le latex inverse ne permet d'obtenir que des textures de type gel-crème. En effet, lorsqu'on tente de préparer à température ambiante une émulsion en utilisant uniquement un latex inverse, il est très difficile d'obtenir des émulsions d'aspect riche et compact.

Lorsque le tensio-actif est le polyglycéryl-6-isostéarate, ce dernier présente l'avantage d'avoir à la fois une partie très hydrophile (grâce aux 6 glycérols) et une partie très hydrophobe (grâce au groupe stéarate). Ces caractéristiques de ce tensio-actif permettent :
- d'accélérer la préparation de la composition cosmétique grâce à la partie hydrophile, et
- de conférer des propriétés crémeuses à la composition cosmétique grâce au groupe stéarate.

La phase grasse de la composition cosmétique préparée selon l'invention peut en outre comprendre au moins un adjuvant cosmétique qui soit utilisable à température ambiante.

Par exemple, la phase grasse de la composition cosmétique peut comprendre au moins un adjuvant choisi parmi :
- les esters gras (par exemple l'isononyl isononanoate, le caprilic capric triglycéride, l'isopropyl myristate),
- les huiles végétales (par exemple l'huile de pépin de raisin, l'huile d'onagre, l'huile de camélia, l'huile de nigelle),
- les huiles minérales,
- les silicones,
- les macérats huileux.

L'isononyl isononanoate est un ester commercialisé par la société SEPPIC sous la dénomination commerciale Lanol® 99.

La phase grasse peut en outre comprendre au moins un principe actif. Il peut s'agir de principes actifs hydratants et émollients (par exemple des acides gras essentiels), anti-vieillissement (par exemple la vitamine E), apaisants (alpha-bisabolol, béta-carotène), séborégulateurs (par exemple l'acide linoléique), amincissants (par exemple les terpènes de *Garcina Cambodgia*)*,* anti-taches (par exemple l'hydroquinone) et antioxydants (par exemple les caroténoïdes).

La phase aqueuse de la composition cosmétique comprend avantageusement de l'eau déminéralisée.

La phase aqueuse peut en outre comprendre au moins un principe actif. Il peut s'agir de principes actifs hydratants et émollients (par exemple la glycérine, sorbitol, les acides aminés), anti-vieillissement (par exemple l'acide hyaluronique, les acides alpha hydroxylés), apaisants (par exemple l'allantoïne), séborégulateurs (par exemple le zinc, le kaolin et les extrait d'hamamélis), amincissants (par exemple la caféine), anti-taches (par exemple l'acide kojique) et antioxydants (par exemple la vitamine C et les flavonoïdes).

Ainsi, la phase grasse et/ou la phase aqueuse peut comprendre au moins un principe actif. Ce principe actif peut être choisi parmi les principes actifs hydratants et émollients, anti-vieillissement, apaisants, séborégulateurs, amincissants, anti-taches et antioxydants.

De manière avantageuse, la phase aqueuse comprend au moins un principe actif qui est un extrait végétal. Par exemple, l'extrait végétal est choisi parmi l'extrait de Fucus, l'extrait d'Aloe Vera, l'extrait de Gingko Biloba, l'extrait de Centella Asiatica, l'extrait de pensée sauvage et l'extrait de Calendula.

De manière préférée, l'extrait végétal comprend de l'eau (avantageusement de l'eau déminéralisée), de la glycérine et au moins un végétal.

De manière avantageuse, l'extrait végétal comprend, en pourcentages massiques par rapport à la masse totale dudit extrait végétal :
- plus de 50% de glycérine, de préférence entre 70% et 90% ;
- entre 10% et 25% d'eau ;
- entre 0,1 et 1% d'au moins un végétal.

La composition cosmétique préparée selon l'invention présente l'avantage que des quantités importantes d'extraits végétaux peuvent être incorporées dans la phase aqueuse sans que cela ne fasse chuter la viscosité de ladite composition, ni sa stabilité au cours du temps.

De manière avantageuse, la teneur massique de l'extrait végétal est d'au plus 20% par rapport à la masse totale de la composition cosmétique préparée selon l'invention.

A la différence des compositions cosmétiques de l'état de l'art, la composition cosmétique préparée selon l'invention peut atteindre une teneur massique en glycérine jusqu'à 10% par rapport à la masse totale de la composition cosmétique préparée selon l'invention, et ce sans qu'elle ne présente une texture collante au toucher ou filante.

La composition cosmétique peut en outre comprendre au moins un parfum.

La composition cosmétique peut en outre comprendre au moins un conservateur. Le conservateur peut par exemple être choisi parmi les parabènes, le phenoxyéthanol et la méthylisothiazolinone.

Dans un mode de réalisation de l'invention, la composition cosmétique comprend au moins :
- un SIMULGEL® EPG ;
- un polyglycéryl-6 isostéarate ;
- une phase aqueuse ;
- une phase grasse qui contient au moins huile végétale et/ou un ester gras;
- optionnellement au moins un parfum et un conservateur.

Dans un mode de réalisation de l'invention, la composition cosmétique comprend au moins :
- un SIMULGEL® EPG ;
- un polyglycéryl-6 isostéarate ;
- une phase aqueuse ;
- une phase grasse qui contient au moins un composé choisi parmi l'huile de pépin de raisin, l'huile d'onagre et l'isononyl isononanoate ;
- optionnellement au moins un parfum et un conservateur.

Dans un mode de réalisation de l'invention, la composition cosmétique comprend au moins :
- un SIMULGEL® EPG;
- un polyglycéryl-6 isostéarate ;
- une phase aqueuse qui contient de l'eau et au moins un extrait végétal, et optionnellement de la glycérine ;
- une phase grasse qui contient au moins huile végétale et/ou un ester gras;
- optionnellement au moins un parfum et un conservateur.

Dans un mode de réalisation de l'invention, la composition cosmétique comprend au moins :
- un SIMULGEL® EPG;
- un polyglycéryl-6 isostéarate ;
- une phase aqueuse qui contient de l'eau et au moins un extrait végétal, et optionnellement de la glycérine ;
- une phase grasse qui contient au moins un composé choisi parmi l'huile de pépin de raisin, l'huile d'onagre et l'isononyl isononanoate ;
- optionnellement au moins un parfum et un conservateur.

Dans un mode de réalisation de l'invention, la composition cosmétique comprend au moins :
- un SIMULGEL® EPG;
- un polyglycéryl-6 isostéarate ;
- une phase aqueuse qui contient de l'eau (de préférence de l'eau déminéralisée) et au moins un extrait végétal, par exemple un extrait végétal choisi parmi l'extrait de Fucus, l'extrait d'Aloe Vera, l'extrait de Gingko Biloba, l'extrait de Centella Asiatica, l'extrait de pensée sauvage et l'extrait de Calendula, et optionnellement de la glycérine ;
- une phase grasse qui contient au moins un principe actif choisi parmi les principes actifs hydratants, anti-vieillissements, apaisants, séborégulateurs, amincissants, dépigmentants, anti-taches, émollients, antioxydants et au moins un composé choisi parmi l'huile de pépin de raisin, l'huile d'onagre et l'isononyl isononanoate ;
- optionnellement au moins un parfum.

La composition cosmétique préparée selon l'invention peut comprendre, en pourcentages massiques exprimés par rapport à la masse totale de ladite composition, au moins :
- entre 0,5% et 5%, de préférence entre 1% et 4%, de latex inverse ;
- entre 0,1% et 10%, de préférence entre 0,5% et 2%, de tensio-actif hydrophile choisi parmi les esters de polyglycérol qui sont liquides à température ambiante et de HLB supérieur ou égal à 8 ;
- entre 70% et 95%, de préférence entre 80% et 85%, de phase aqueuse ;
- entre 1% et 20%, de préférence entre 3% et 6%, de phase grasse ;
- optionnellement entre 0,1% et 0,3% de parfum ;
- optionnellement entre 0,1% et 1% de conservateur.

Selon les quantités de ses différents constituants, la composition cosmétique sous forme d'une émulsion huile dans eau préparée selon l'invention peut être une crème, un gel crèmeux, un fluide ou encore un sérum.

L'invention a ainsi pour objet un procédé de préparation de la composition cosmétique telle que décrite ci-dessus qui comprend au moins les étapes suivantes :
a) on prépare un 1^{ier} mélange comprenant au moins une phase grasse, un latex inverse et un tensio-actif hydrophile qui est choisi parmi les esters de polyglycérol qui sont liquides à température ambiante et de HLB supérieur ou égal à 8 ;
b) on ajoute à ce 1^{ier} mélange une phase aqueuse de manière à obtenir un 2^{ième} mélange ;
c) optionnellement, si dans le 2^{ième} mélange à l'issue de l'étape b) aucune émulsion huile dans eau ne s'est formée spontanément, on homogénéise ce 2^{ième} mélange jusqu'à l'obtention d'une émulsion huile dans eau de ladite composition cosmétique,
le rapport du pourcentage massique dudit tensio-actif sur le pourcentage massique dudit latex inverse étant compris entre 0,1 et 1, plus préférentiellement compris entre 0,15 et 0,6, lesdits pourcentages massiques étant exprimés par rapport à la masse totale de ladite composition cosmétique.

Dans un mode de réalisation de l'invention, toutes les étapes du procédé sont réalisées à température ambiante.

Dans un mode de réalisation du procédé de préparation, l'étape a) peut être réalisée de la manière suivante :
- on disperse le tensio-actif hydrophile choisi parmi les esters de polyglycérol qui sont liquides à température ambiante et de HLB supérieur ou égal à 8 dans la phase grasse, puis
- on ajoute le latex inverse.

Dans un autre mode de réalisation du procédé de préparation, on peut tout d'abord disperser le latex inverse dans la phase grasse, puis y ajouter le tensio-actif.

A l'étape a), l'ordre d'incorporation des différents constituants n'a pas d'importance.

Au cours de l'étape a), dans le 1^{ier} mélange qui comprend donc notamment la phase grasse, le tensio-actif déclenche l'inversion de phases du latex inverse. Le tensio-actif amorce au sein de la phase grasse le déploiement du polymère du latex inverse. Ce déploiement est poursuivi, de manière amplifiée, lors de l'ajout de la phase aqueuse. L'amorçage du déploiement du polymère dans la phase grasse avant l'ajout de la phase aqueuse se révèle très bénéfique pour la vitesse de fabrication de la composition selon l'invention.

De manière avantageuse, à l'étape b), on ajoute lentement la phase aqueuse.

Au cours de l'étape b), on peut agiter le 1^{ier} mélange en même temps que l'on y ajoute la phase aqueuse.

Cette agitation n'est cependant pas indispensable pour l'obtention de l'émulsion de la composition cosmétique. Cela est avantageux pour la personne qui prépare la composition cosmétique qui n'est pas contrainte d'agiter en continu le mélange en vue d'obtenir une émulsion. Ainsi, le procédé de préparation présente l'avantage d'être particulièrement facile à mettre en œuvre et nécessite peu d'actions de la part de cette personne.

Dès lors qu'on ajoute la phase aqueuse, le polymère du latex inverse dont le déploiement a été amorcé dans la phase grasse grâce à la présence du tensio-actif s'hydrate et continue ainsi à se déployer.

A l'étape b), lorsqu'on ajoute la phase aqueuse dans le 1^{ier} mélange, la phase grasse commence à blanchir. Cela signifie que l'émulsion huile dans eau de la composition cosmétique commence à se former spontanément, et ce sans une étape c) d'homogénéisation.

De manière avantageuse, une fois la phase aqueuse ajoutée, on peut laisser le polymère s'hydrater en laissant au repos la composition cosmétique en cours de préparation.

Lorsque la totalité de la phase aqueuse a été ajoutée et au cours de la phase de repos, on observe à l'œil nu des filaments fins qui se déploient. Il s'agit du polymère du latex inverse qui s'hydrate.

Lorsqu'on constate qu'il n'y a plus de phase aqueuse libre (à savoir qu'il n'y a plus de phase aqueuse qui surnage) du fait de l'hydratation du polymère, on peut éventuellement homogénéiser avec un fouet de cuisine ou une spatule si l'émulsion huile dans eau ne s'est pas formée spontanément et totalement à l'issue de l'ajout de la phase aqueuse.

Dans certains cas de préparation de la composition cosmétique, l'émulsion huile dans eau va se former spontanément, et ce sans avoir à procéder à une homogénéisation.

Le tensio-actif diminue la tension de surface de façon telle que l'émulsion se forme quasi-spontanément, voire spontanément, et demeure stable au cours du temps.

Le tensio-actif se situe à l'interface des globules de la phase grasse dispersée dans la phase aqueuse. Ces globules de la phase grasse sont fins et répartis de manière homogène ; ce qui stabilise l'émulsion obtenue à l'issue du procédé de préparation selon l'invention.

Avec le procédé de préparation selon l'invention, l'émulsion se forme à température ambiante et très rapidement, en moins de 10 minutes, voire même moins de 5 minutes. Le procédé de préparation présente ainsi l'avantage ne pas nécessiter d'agitation mécanique mise en œuvre avec un appareillage électrique. L'émulsion se forme quasi-spontanément en homogénéisant (par exemple à l'aide d'une spatule), voire même parfois spontanément selon la nature et la quantité des constituants de la composition cosmétique qui ont été décrits ci-dessus, et ce grâce à la synergie du tensio-actif et du latex inverse.

L'absence d'étapes de chauffage, de refroidissement et la non nécessité d'appareillages complexes pour l'agitation sont particulièrement avantageux pour préparer la composition cosmétique dans le cadre d'un usage domestique qui a été mentionné ci-dessus.

En outre, l'absence de chauffage et d'agitation mécanique vigoureuse au cours du procédé de préparation selon l'invention préserve parfaitement les principes actifs sensibles à la chaleur. C'est pourquoi, la composition cosmétique préparée selon l'invention peut comprendre des principes actifs très variés, et notamment des principes actifs sensibles à la chaleur. Cela constitue un autre avantage de la composition cosmétique préparée selon l'invention.

Enfin, l'absence de ces étapes de chauffage et d'agitation mécanique à l'aide d'appareillages électriques confère également l'avantage au procédé de préparation selon l'invention d'être particulièrement économique d'un point de vue énergétique.

A l'étape b) du procédé de préparation, lors de l'ajout de la phase aqueuse dans le 1^{ier} mélange, la présence du tensio-actif hydrophile choisi parmi les esters de polyglycérol qui sont liquides à température ambiante et de HLB supérieur ou égal à 8 tel que par exemple le polyglycéryl-6 isostéarate, permet au latex inverse de se déployer plus facilement et favorise la formation d'un microgel dans la phase continue de l'émulsion. En l'absence d'un tel tensio-actif, ledit latex formerait un amas dense de gel qui se déploierait difficilement lors de l'ajout de la phase aqueuse. Lorsque ledit tensio-actif a été dispersé dans la phase grasse, on ne constate pas un tel amas constitué du latex inverse.

Le procédé de préparation de la composition cosmétique présente en outre l'avantage qu'il peut être mis en œuvre avec un seul récipient. En effet, on peut préparer le 1^{ier} mélange de l'étape a) en pesant et en ajoutant successivement dans le recipient les différents constituants que sont la phase grasse, le tensio-actif et le latex inverse, et ensuite à l'étape b) en y ajoutant la phase aqueuse dans ledit récipient. L'utilisation d'un seul récipient pour réaliser la composition cosmétique est particulièrement avantageuse si l'on destine la préparation de cette composition à un cadre domestique.

La présente invention a également pour objet un kit de préparation d'une composition cosmétique telle que décrite ci-dessus, ledit kit comprend au moins :
- un 1^{ier} récipient contenant au moins la phase aqueuse,
- un 2^{ième} recipient contenant au moins la phase grasse, le latex inverse et le tensio-actif hydrophile qui est choisi parmi les esters de polyglycérol qui sont liquides à température ambiante et de HLB supérieur ou égal à 8.

Ledit kit peut comprendre un 3^{ième} récipient dans lequel on va préparer la composition cosmétique, et ce comme cela a été décrit ci-dessus.

Ledit kit peut en outre comprendre un dispositif de pesée.

Ledit kit peut également comprendre au moins un ustensile de mélange, par exemple un fouet de cuisine ou une spatule (en bois ou en plastique).

Dans un mode de réalisation de l'invention, ledit kit comprend en outre une notice explicative de la préparation de la composition cosmétique.

Les phases grasse et aqueuse, le latex inverse et le tensio-actif du kit selon l'invention ont été détaillés ci-dessus dans la description de la composition cosmétique préparée selon l'invention.

### PARTIE EXPERIMENTALE :

Afin de démontrer l'effet synergique entre le latex inverse et le tensio-actif hydrophile choisi parmi les esters de polyglycérol qui sont liquides à température ambiante et de HLB supérieur ou égal à 8 dans la composition cosmétique, les expérimentations détaillées ci-dessous ont été mises en œuvre.

### I - Effet de la présence du tensio-actif dans la composition cosmétique sur son temps de préparation :

Une 1^{ière} expérimentation a consisté à déterminer l'influence du tensio-actif dans la composition cosmétique préparée selon l'invention (« composition selon l'invention ») sur son temps de préparation en comparaison avec une composition cosmétique équivalente dite « comparative », car dépourvue de ce tensio-actif.

Le polyglycéryl-6 isostéarate (ci-après abrégé « Plurol ») est le tensio-actif qui a été testé.

Le tableau 1 ci-dessous détaille :
- les pourcentages massiques par rapport à la masse totale de la composition considérée des constituants de :
   ∘ la composition comparative ;
   ∘ la composition préparée selon l'invention libellée "composition selon l'invention" dans le tableau 1 ;
- le temps de préparation total de la composition considérée comprenant :
   ∘ le temps d'hydratation (c'est-à-dire le temps pendant lequel on a laissé reposer la composition, à savoir le temps entre la fin de l'ajout de la phase aqueuse et le début de l'homogénéisaton), suivi
   ∘ du temps d'homogénéisation (à savoir le temps jusqu'à l'obtention d'une émulsion stable d'aspect lisse et homogène).

**Tableau 1 détaillant les constituants des compositions comparative et préparée selon l'invention, ainsi que les temps de préparation respectifs**

| Phases | Constituants | Composition comparative | Composition selon l'invention |
|---|---|---|---|
| A | Eau déminéralisée | QSP 100% | QSP 100% |
| B | Conservateur | 0,4% (phenoxyethanol) | 2% |
| | Corps gras | 3% (huile de pépin de raisin : 1% et isononyl isononanoate : 2%) | 3% |
| | SIMULGEL® EPG | 4% | 4% |
| | Plurol | 0% | 2% |
| Temps de préparation | | 5 minutes 35 secondes | 1 minute 10 secondes |
| Temps d'hydratation | | 4 minutes 35 secondes | 45 secondes |
| Temps d'homogénéisation | | 1 minute | 25 secondes |

Dans le tableau 1 ci-dessus et les autres tableaux qui suivent, « QSP » est l'acronyme de «Quantité suffisante pour» pour signifier que le pourcentage massique d'eau déminéralisée dans la composition est tel qu'additionné aux pourcentages de tous les autres constituants de ladite composition, on obtienne un total de 100%.

Les compositions ont été réalisées de la manière suivante :
On a pesé séparément les constituants des phases A et B.

Pour la phase B, on a pesé d'abord le SIMULGEL® EPG, puis on a ajouté les autres constituants de cette phase B et on a homogénéisé avant d'y incorporer la totalité de l'eau déminéralisée (c'est-à-dire la phase A) en une fois.

Après avoir ajouté l'eau déminéralisée, on a laissé le polymère du SIMULGEL® EPG s'hydrater (ce qui correspond au temps d'hydratation). Puis on a agité doucement au fouet jusqu'à l'obtention d'une émulsion (ce qui correspond au temps d'homogénéisation).

Au vu du tableau 1, on relève que la présence du Plurol augmente de manière très signitificative la vitesse d'hydratation et favorise l'homogénisation.

L'émulsion de la composition cosmétique préparée selon l'invention est obtenue de manière beaucoup plus rapide que celle de la composition comparative.

### Il - Effet de la quantité du tensio-actif dans la composition cosmétique sur son temps de préparation :

Une 2^{ième} expérimentation a consisté à déterminer l'influence de la quantité de tensio-actif dans la composition cosmétique préparée selon l'invention (« composition selon l'invention ») sur son temps de préparation et également en comparaison avec une composition cosmétique équivalente dite « comparative », car dépourvue de ce tensio-actif.

Le tableau 2 ci-dessous détaille les pourcentages massiques par rapport à la masse totale de la composition considérée des constituants :
∘ d'une composition comparative (« Essai 1 ») ;
∘ de compositions préparées selon l'invention (Essais 2 à 6 ») dans lesquelles le pourcentage massique de Plurol varie entre 0,15% et 1,2% ;
   - le temps de préparation total de la composition considérée comprenant :
∘ le temps d'hydratation, suivi
∘ du temps d'homogénéisation.

Les compositions ont été réalisées de la même manière que dans la 1^{ière} expérimentation.

Le corps gras et le conservateur indiqués dans le tableau 2 sont les mêmes que ceux détaillés dans le tableau 1 ci-dessus.

**Tableau 2 détaillant les constituants des compositions comparative (Essai 1) et préparée selon l'invention (Essais 2 à 6), ainsi que les temps de préparation respectifs**

| Phase | Constituants | Essai 1 | Essai 2 | Essai 3 | Essai 4 | Essai 5 | Essai 6 |
|---|---|---|---|---|---|---|---|
| A | Eau déminéralisé e | QSP 100% | QSP 100% | QSP 100% | QSP 100% | QSP 100% | QSP 100% |
| B | Conservateur | 2% | 2% | 2% | 2% | 2% | 2% |
| | Corps gras | 3% | 3% | 3% | 3% | 3% | 3% |
| | SIMULGEL® EPG | 1% | 1% | 1% | 1% | 1% | 1% |
| | Plurol | 0% | 0,15% | 0,35% | 0,5% | 1% | 1,2% |
| Temps de réalisation | | Supérieur à 15 minutes | 7 minutes | 5 minutes | 5 minutes | 5 minutes | 5 minutes |
| Temps d'hydratation | | | 5 minutes | 4 minutes | 4 minutes | 4 minutes | 4 minutes |
| Temps d'homogénéisation | | | 2minutes | 1 minute | 1 minute | 1 minute | 1 minute |

Au vu du tableau 2, on relève qu'à partir d'un rapport du pourcentage massique du Plurol sur celui du SIMULGEL® EPG de 0,35, la vitesse de préparation est optimale : elle demeure constante à 5 minutes.

En outre, plus le pourcentage de Plurol augmente, plus la texture devient crémeuse, riche et épaisse.

### III - Effet de la quantité du latex inverse dans la composition cosmétique sur son temps de préparation :

Une 3^{ième} expérimentation a consisté à déterminer l'influence de la quantité du latex inverse dans des compositions cosmétiques préparées selon l'invention sur son temps de préparation.

Au cours de cette 3^{ième} expérimentation, le rapport du pourcentage massique du tensio-actif sur celui du latex était toujours de 0,5.

Le tableau 3 ci-dessous détaille :
- les pourcentages massiques par rapport à la masse totale de la composition considérée des constituants de 4 compositions préparées selon l'invention (Essais 1 à 4 ») dans lesquelles le pourcentage massique de SIMULGEL® EPG varie entre 1% et 4%.
- le temps de préparation total de la composition considérée incluant :
   ∘ le temps d'hydratation, suivi
   ∘ du temps d'homogénéisation.

Les compositions ont été réalisées de la même manière que dans la 1^{ière} expérimentation.

Le corps gras et le conservateur indiqués dans le tableau 3 sont les mêmes que ceux détaillés dans le tableau 1 ci-dessus.

**Tableau 3 détaillant les constituants des compositions préparées selon l'invention (Essais 1 à 4), ainsi que les temps de préparation respectifs**

| Phases | Constituants | Essai 1 | Essai 2 | Essai 3 | Essai 4 |
|---|---|---|---|---|---|
| A | Eau déminéralisée | QSP 100% | QSP 100% | QSP 100% | QSP 100% |
| B | Conservateur | 2% | 2% | 2% | 2% |
| | Corps gras | 3% | 3% | 3% | 3% |
| | SIMULGEL® EPG | 1% | 2% | 3% | 4% |
| | Plurol | 0,5% | 1% | 1,5% | 2% |
| Temps de préparation | | 5 minutes | 4 minutes | 3 minutes | 1 minute 10 secondes |
| Temps d'hydratation | | 4 minutes | 3 minutes | 2 minutes | 45 secondes |
| Temps d'homogénéisation | | 1 minute | 1 minute | 1 minute | 25 secondes |

Au vu du tableau 3, on relève que plus la quantité de SIMULGEL® EPG augmente, plus la fabrication de la composition cosmétique selon l'invention est rapide car la phase d'hydratation est rapide. Pour 4% de SIMULGEL® EPG, l'émulsion se forme quasiment spontanément.

### IV - Effet du tensio-actif dans la composition cosmétique sur sa stabilité lorsque la texture de la composition cosmétique est épaisse

Une 4^{ième} expérimentation a consisté à déterminer l'influence du tensio-actif sur la stabilité d'une composition cosmétique préparée selon l'invention de texture épaisse (6% de corps gras) en comparaison avec celle d'une composition équivalent dite « comparative », car dépourvue de tensio-actif.

Le tableau 4 ci-dessous détaille :
- les pourcentages massiques par rapport à la masse totale de la composition considérée des constituants de :
   ∘ la composition comparative (« Essai 1 ») ;
   ∘ la composition préparée selon l'invention (« Essai 2 »);
- la stabilité observée.

Les compositions ont été réalisées de la même manière que dans la 1^{ière} expérimentation.

**Tableau 4 détaillant les constituants des compositions comparative (Essai 1) et selon l'invention (Essai 2), ainsi que les stabilités respectives observées**

| Phases | Constituants | Essai 1 | Essai 2 |
|---|---|---|---|
| A | Eau déminéralisée | QSP 100% | QSP 100% |
| B | Conservateur | 2% | 2% |
| | Corps gras | 6% (4 % d'huile de raisin et 2% d'isononyl) | 6% |
| | SIMULGEL® EPG | 4% | 4% |
| | Plurol | 0% | 2% |
| Stabilité | | Début de déphasage observé après 1 mois à 45°C | Stable 1 mois à 45°C |

Au vu du tableau 4, on relève que l'émulsion de la composition cosmétique demeure parfaitement stable au cours du temps, et ce même à une température élevée de 45°C ; ce qui n'est pas le cas de l'émulsion comparative qui déphase à cette température.

La composition cosmétique préparée selon l'invention présente l'avantage de rester parfaitement stable au cours du temps.

### V - Effet du tensio-actif dans la composition cosmétique sur sa texture et son apparence

Une 5^{ième} expérimentation a consisté à déterminer l'influence du tensio-actif sur la texture et la richesse au toucher de 2 compositions cosmétiques préparées selon l'invention en comparaison avec une composition équivalente dite « comparative », car dépourvue de tensio-actif.

Le tableau 5 ci-dessous détaille :
- les pourcentages massiques par rapport à la masse totale de la composition considérée des constituants :
   ∘ de la composition comparative (« Essai 1 ») ;
   ∘ des compositions préparées selon l'invention (« Essais 2 et 3»);
- la texture observée ;
- la richesse au toucher ressentie.

La richesse au toucher a été évaluée par 5 personnes qui ont jugé les compositions testées en indiquant dans quelle mesure elles trouvaient que la composition s'étalait correctement. Plus précisément, les critères évalués par ces 5 personnes étaient la facilité d'étalement sur la peau, la sensation de douceur au toucher après application et la sensation d'effet collant ou non. Une richesse au toucher « bonne » ou « excellente » signifie que les personnes ont trouvé que l'émulsion testée s'étalait bien sur la peau tout en présentant un caractère très onctueux et était agréable au toucher une fois appliquée.

Les compositions ont été réalisées de la même manière que dans la 1^{ière} expérimentation.

Le corps gras et le conservateur indiqués dans le tableau 5 sont les mêmes que ceux détaillés dans le tableau 1 ci-dessus.

**Tableau 5 détaillant les constituants de la composition comparative (Essai 1) et des compositions préparées selon l'invention (Essai 2 et 3), ainsi que leur texture observée et leur richesse au toucher appréciée**

| Phases | Constituants | Essai 1 | Essai 2 | Essai 3 |
|---|---|---|---|---|
| A | Eau déminéralisée | QSP 100% | QSP 100% | QSP 100% |
| B | Conservateur | 2% | 2% | 2% |
| | Corps gras | 3% | 3% | 3% |
| | SIMULGEL® EPG | 4% | 4% | 4% |
| | Plurol | 0% | 2% | 4% |
| Texture | | Gel opalescent compact | Emulsion compacte | Emulsion compacte et crémeuse |
| Richesse au toucher | | Très mauvaise | Bonne | Excellente |

Au vu du tableau 5, on relève que les émulsions des compositions cosmétiques préparées selon l'invention présentent une texture compacte et crémeuse, ainsi que de très bonnes sensations de richesse au toucher, et ce à la différence de la composition comparative. Il n'y a aucune sensation de richesse et d'onctuosité au toucher de l'émulsion de l'essai 1. Il s'agit d'une gelée fraîche au toucher aqueux.

### VI - Effet du tensio-actif dans la composition cosmétique lorsqu'elle comprend d'importantes quantités d'extraits végétaux :

Une 6^{ième} expérimentation a consisté à déterminer l'influence du tensio-actif sur la texture d'une composition cosmétique préparée selon l'invention riche en extrait végétal (10% de la masse totale de la composition) en comparaison avec celle d'une composition équivalente dite comparative car dépourvue de tensio-actif.

Le tableau 6 ci-dessous détaille :
- les pourcentages massiques par rapport à la masse totale de la composition considérée des constituants de :
   ∘ la composition comparative (« Essai 1 ») ;
   ∘ la composition préparée selon l'invention (« Essai 2»);
- le temps de préparation total de la composition considérée incluant :
   ∘ le temps d'hydratation (, suivi
   ∘ du temps d'homogénéisation.
- la texture observée.

Les compositions ont été réalisées de la même manière que dans la 1^{ière} expérimentation.

Le corps gras et le conservateur indiqués dans le tableau 6 sont les mêmes que ceux détaillés dans le tableau 1 ci-dessus.

Les compositions comprenaient 10% en masse d'extraits végétaux. Ces 10% extraits végétaux étaient composés de : 2% d'extrait d'Aloe Vera, 2% d'extrait de Calendula, 2% d'extrait de Centella Asiatica, 2% d'extrait de Ginkgo Biloba et de 2% Extrait de Pensée Sauvage 2%.

**Tableau 6 détaillant les constituants de la composition comparative (Essai 1) et de la composition préparée selon l'invention (Essai 2), ainsi que leur temps de préparation et texture respectives**

| Phases | Constituants | Essai 1 | Essai 2 |
|---|---|---|---|
| A | Eau | QSP 100% | QSP 100% |
| | Extraits végétaux | 10% | 10% |
| B | Conservateur | 2% | 2% |
| | Corps gras | 3% | 3% |
| | SIMULGEL® EPG | 4% | 4% |
| | Plurol | 0% | 2% |
| Temps de préparation | | 10 minutes | 1 minute 10 secondes |
| Temps d'hydratation | | 5 minutes | 45 secondes |
| Temps d'homogénéisation | | 5 minutes sous agitation vive | 25 secondes |
| Texture | | Gel jaunâtre | Crème épaisse couleur pêche |

Au vu du tableau 6, on relève que l'émulsion de la composition cosmétique préparée selon l'invention se forme beaucoup plus rapidement que la composition comparative et qu'elle présente en outre un aspect crémeux et compact qui correspond parfaitement aux textures recherchées dans le domaine cosmétique. La texture de la composition cosmétique préparée selon l'invention est bien différente de celle de la composition comparative qui se présente sous forme d'un gel jaunâtre.

Cette expérimentation témoigne de la capacité des compositions cosmétiques préparées selon l'invention à incorporer des quantités importantes d'extraits végétaux sans que cela ne porte préjudice à sa texture crémeuse et compacte.

### VII - Effet du tensio-actif dans la composition cosmétique sur son temps de préparation et sa richesse au toucher :

Une 7^{ième} expérimentation a consisté à déterminer l'influence du tensio-actif dans la composition cosmétique préparée selon l'invention (« composition selon l'invention ») sur son temps de préparation et sa texture en comparaison avec une composition cosmétique équivalente dite « comparative », car dépourvue de ce tensio-actif.

Le tableau 7 ci-dessous détaille :
- les pourcentages massiques par rapport à la masse totale de la composition considérée des constituants de :
   ∘ la composition préparée selon l'invention libellée "composition selon l'invention" dans le tableau 7 ;
   ∘ la composition comparative ;
- le temps de préparation total de la composition considérée comprenant :
   ∘ le temps d'hydratation, suivi
   ∘ du temps d'homogénéisation.

Les compositions ont été réalisées de la même manière que dans la 1^{ière} expérimentation, à la différence que le latex inverse était le SIMULGEL® NS et non pas le SIMULGEL® EPG.

Le corps gras et le conservateur indiqués dans le tableau 7 sont les mêmes que ceux détaillés dans le tableau 1 ci-dessus.

**Tableau 7 détaillant les constituants des compositions comparative et préparée selon l'invention, ainsi que les temps de préparation respectifs et leur richesse au toucher**

| Phases | Constituants | Composition selon l'invention | Composition comparative |
|---|---|---|---|
| A | Eau déminéralisée | QSP 100% | QSP 100% |
| B | Conservateur | 2% | 2% |
| | Corps gras | 3% | 3% |
| | SIMULGEL® NS | 3% | 3% |
| | Plurol | 1,5% | 0% |
| Temps de préparation | | 1 minute 33 secondes | 3 minutes |
| Temps d'hydratation | | 1 minute | 2 minutes |
| Temps d'homogénéisation | | 33 secondes | 1 minute |
| Richesse au toucher | | Très mauvaise | Excellente |

Au vu du tableau 7, de même qu'avec le SIMULGEL® EPG, on relève que la présence du Plurol a un impact très positif sur la vitesse de fabrication et la richesse au toucher des émulsions obtenues.

L'émulsion de la composition cosmétique préparée selon l'invention est obtenue beaucoup plus rapidement que celle de la composition comparative et présente une excellente richesse au toucher ; ce qui n'est pas le cas de la composition comparative.

### VIII - Préparation de compositions selon l'invention avec d'autres tensio-actifs que le Plurol :

Une 8^{ième} expérimentation a consisté à préparer des compositions selon l'invention avec d'autres tensio-actifs que le Plurol.

Les 2 autres tensio-actifs testés étaient :
- le polyglycéryl-4 oleate de HLB égal à 10 ;
- le polyglycéryl-4 oleate de HLB égal à 8.

Ces 2 tensio-actifs sont proches du Plurol car ils ont une taille de chaîne carbonée équivalente, mais un nombre de glycérols estérifiés inférieur.

Le tableau 8 ci-dessous détaille :
- les pourcentages massiques par rapport à la masse totale de la composition considérée des constituants de 3 compositions préparées selon l'invention.
- le temps de préparation total de la composition considérée comprenant :
   ∘ le temps d'hydratation, suivi
   ∘ du temps d'homogénéisation,
- l'aspect final visuel de l'émulsion,
- la richesse au toucher.

Les compositions ont été réalisées de la même manière que dans la 1^{ière} expérimentation, à la différence que le Plurol pour l'essai 2 a été remplacé par le polyglycéryl-4 oléate de HLB égal à 10 et pour l'essai 3, il a été remplacé par le polyglycéryl-4 oléate de HLB égal à 8.

Le corps gras et le conservateur indiqués dans le tableau 8 sont les mêmes que ceux détaillés dans le tableau 1 ci-dessus.

**Tableau 8 détaillant les constituants des compositions préparées selon l'invention, ainsi que les temps de préparation respectifs et leur richesse au toucher**

| Phases | Constituants | Essai 1 | Essai 2 | Essai 3 |
|---|---|---|---|---|
| A | Eau déminéralisée | QSP 100% | QSP 100% | QSP 100% |
| B | Conservateur | 2% | 2% | 2% |
| | Corps gras | 3% | 3% | 3% |
| | SIMULGEL® EPG | 3% | 3% | 3% |
| | Plurol | 1,5% | 0% | 0% |
| | Polyglyceryl-4 Oleate (HLB 10) | 0% | 1,5% | 0% |
| | Polyglyceryl-4 Oleate (HLB 8) | 0% | 0% | 1,5% |
| Temps de préparation | | 3 minutes | 4 minutes | 4 minutes |
| Temps d'hydratation | | 2 minutes 30 secondes | 3 minutes | 3 minutes |
| Temps d'homogénéisation | | 30 secondes | 1 minute | 1 minute |
| Aspect final visuel | | Aspect ultra crémeux | Aspect gel-crème (peu crémeux) | Aspect crémeux |
| Richesse au toucher | | Excellente | Bonne | Très bonne |

Au vu du tableau 8, on relève que ces compositions cosmétiques préparées selon l'invention mises en œuvre avec un autre tensio-actif que le Plurol sont préparées rapidement et présentent un aspect final visuel et une richesse au toucher tout à fait acceptables, voire même excellents.

### IX - Préparation d'une composition dont le tensio-actif a un HLB inférieur à 8 :

Une 9^{ième} expérimentation a consisté à préparer une composition comparative par rapport aux compositions préparées selon l'invention, car le tensio-actif avait un HLB de 1,5.

Le tensio-actif utilisé était un polyglycéride des acides linolénique, linoléique et oléique commercialisé par la société ALDIVIA sous la dénomination commerciale Viamerine®2500.

Le tableau 9 ci-dessous détaille :
- les pourcentages massiques par rapport à la masse totale de la composition,
- le temps de préparation de la composition,
- la richesse au toucher,
- la stabilité

La composition a été réalisée de la même manière que dans la 1^{ière} expérimentation, à la différence que le Plurol a été remplacé par Viamerine®2500.

Le corps gras et le conservateur indiqués dans le tableau 9 sont les mêmes que ceux détaillés dans le tableau 1 ci-dessus.

**Tableau 9 détaillant les constituants de la composition comparative, sa richesse au toucher et sa stabilité**

| Phases | Constituants | Essai 1 |
|---|---|---|
| A | Eau déminéralisée | QSP 100% |
| B | Conservateur | 2% |
| | Corps gras | 3% |
| | SIMULGEL® EPG | 3% |
| | Viamerine® 2500 | 3% |
| Temps de préparation | | Supérieur à 20 minutes avec agitation mécanique |
| Aspect final visuel | | Très riche et onctueux |
| Stabilité | | Déphasage au bout de 24 heures |

Au vu du tableau 9, on relève que la composition comparative est difficilement préparable car le temps de préparation est long et nécessite une agitation mécanique. De plus, à la différence des compositions cosmétiques préparées selon l'invention, cette composition n'est pas stable. Au bout de 24 heures, elle a déjà déphasé. Cette expérimentation témoigne de l'importance de la sélection d'un tensio-actif appropriée, à savoir qui présente notamment un HLB supérieur ou égal à 8, pour préparer rapidement et aisément des compositions cosmétiques selon le procédé de l'invention qui soient stables au cours du temps.

## Revendications

1. Procédé de préparation d'une composition cosmétique sous forme d'une émulsion huile dans eau, **caractérisé en ce qu'**il comprend au moins les étapes suivantes :
a) on prépare un 1^{ier} mélange comprenant au moins une phase grasse, un latex inverse et un tensio-actif hydrophile qui est choisi parmi les esters de polyglycérol qui sont liquides à température ambiante et de HLB (« HLB » étant l'acronyme anglophone de « Hydrophilic-Lipophilic Balance » se traduisant par « équilibre hydrophile/lipophile ») supérieur ou égal à 8 ;
b) on ajoute à ce 1^{ier} mélange une phase aqueuse de manière à obtenir un 2^{ième} mélange ;
c) optionnellement, si dans le 2^{ième} mélange à l'issue de l'étape b) aucune émulsion huile dans eau ne s'est formée spontanément, on homogénéise ce 2^{ième} mélange jusqu'à l'obtention d'une émulsion huile dans eau de ladite composition cosmétique,
le rapport du pourcentage massique dudit tensio-actif sur le pourcentage massique dudit latex inverse étant compris entre 0,1 et 1, plus préférentiellement compris entre 0,15 et 0,6, lesdits pourcentages massiques étant exprimés par rapport à la masse totale de ladite composition cosmétique.

2. Procédé de préparation selon la revendication 1, **caractérisé en ce que** toutes ses étapes sont réalisées à température ambiante.

3. Procédé de préparation selon la revendication 1 ou 2, **caractérisé en ce que** l'ester de polyglycérol est choisi dans le groupe constitué par polyglycéryl-2 caprate, polyglycéryl-2 laurate, polyglycéryl-3 laurate, polyglycéryl-4 laurate, polyglycéryl-4 isostéarate, polyglycéryl-4 oléate, polyglycéryl-5 laurate, polyglycéryl-5 oléate, polyglycéryl-5 dioléate, polyglycéryl-6 caprylate, polyglycéryl-6 tricaprylate, polyglycéryl-10 laurate, polyglycéryl-10 myristate, polyglycéryl-10 isostéarate, polyglycéryl-10 diisostéarate, polyglycéryl-10 oléate et polyglycéryl-6-isostéarate.

4. Procédé de préparation selon la revendication 3, **caractérisé en ce que** le tensio-actif est le polyglycéryl-6 isostéarate.

5. Procédé de préparation selon l'une quelconque des revendications 1 à 4, **caractérisé en ce que** ladite composition cosmétique comprend, en pourcentages massiques exprimés par rapport à la masse totale de ladite composition, au moins :
- entre 0,5% et 5%, de préférence entre 1% et 4%, de latex inverse ;
- entre 0,1% et 10%, de préférence entre 0,5% et 2%, de tensio-actif hydrophile choisi parmi les esters de polyglycérol qui sont liquides à température ambiante et de HLB supérieur ou égal à 8 ;
- entre 70% et 95%, de préférence entre 80% et 85%, de phase aqueuse ;
- entre 1% et 20%, de préférence entre 3% et 6%, de phase grasse ;
- optionnellement entre 0,1% et 0,3%, de parfum ;
- optionnellement entre 0,1% et 1%, de conservateur.

6. Procédé de préparation selon l'une quelconque des revendications 1 à 5, **caractérisé en ce que** la phase grasse comprend au moins un adjuvant choisi parmi les esters gras, les huiles végétales, les huiles minérales, les silicones et les macérats huileux.

7. Procédé de préparation selon l'une quelconque des revendications 1 à 6, **caractérisé en ce que** la phase grasse et/ou la phase aqueuse comprend au moins un principe actif qui est choisi parmi les principes actifs hydratants et émollients, anti-vieillissement, apaisants, séborégulateurs, amincissants, anti-taches et antioxydants.

8. Procédé de préparation selon l'une quelconque des revendications 1 à 7, **caractérisé en ce que** la phase aqueuse comprend au moins un principe actif qui est un extrait végétal choisi parmi l'extrait de Fucus, l'extrait d'Aloe Vera, l'extrait de Gingko Biloba, l'extrait de Centella Asiatica, l'extrait de pensée sauvage et l'extrait de Calendula.

9. Procédé de préparation selon l'une quelconque des revendications 1 à 8, **caractérisé en ce que** ladite composition cosmétique est une crème, un gel crémeux, un fluide ou un sérum.

10. Kit de préparation d'une composition cosmétique préparée selon le procédé selon l'une quelconque des revendications 1 à 9 qui comprend au moins :
- un 1^{ier} récipient contenant au moins la phase aqueuse,
- un 2^{ième} recipient contenant au moins la phase grasse, le latex inverse et le tensio-actif hydrophile qui est choisi parmi les esters de polyglycérol qui sont liquides à température ambiante et de HLB supérieur ou égal à 8.

## Patentansprüche

1. Verfahren zur Zubereitung einer kosmetischen Zusammensetzung in Form einer Öl-in-Wasser-Emulsion, **dadurch gekennzeichnet, dass** es wenigstens die folgenden Schritte umfasst:
a) Herstellen der 1. Mischung, umfassend wenigstens eine Fettphase, ein inverses Latex und ein hydrophiles Tensid, das ausgewählt ist aus den Polyglyerolestern, die bei Raumtemperatur flüssig sind, und aus HLB ("HLB" ist die englische Abkürzung von "Hydrophilic-Lipophilic Balance", was mit "Hydrophil-Lipophil-Gleichgewicht" übersetzbar ist) höher als oder gleich 8;
b) Hinzufügen einer wässrigen Phase zu der 1. Mischung, sodass eine 2. Mischung gewonnen wird;
c) optional, wenn sich in der 2. Mischung durch den Schritt b) spontan keine Öl-Emulsion in dem Wasser bildet, Homogenisieren der 2. Mischung bis zur Gewinnung einer Öl-in-Wasser-Emulsion der kosmetischen Zusammensetzung,
wobei das Verhältnis des Massenanteils des Tensids zu dem Massenanteil des inversen Latex zwischen 0,1 und 1 beträgt, bevorzugter zwischen 0,15 und 0,6, wobei die Massenanteile ausgedrückt werden durch das Verhältnis des Gesamtgewichts zu der kosmetischen Zusammensetzung.

2. Herstellungsverfahren nach Anspruch 1, **dadurch gekennzeichnet, dass** alle seine Schritte bei Raumtemperatur durchgeführt werden.

3. Herstellungsverfahren nach Anspruch 1 oder 2, **dadurch gekennzeichnet, dass** das Polyglyerolester ausgewählt ist aus der Gruppe bestehend aus Polyglyceryl-2 Caprate, Polyglyceryl-2 Laurate, Polyglyceryl-3 Laurate, Polyglyceryl-4 Laurate, Polyglyceryl-4 Isostearate, Polyglyceryl-4 Isostearate, Polyglyceryl-4 Oleate, Polyglyceryl-5 Laurate, Polyglyceryl-5 Oleate, Polyglyceryl-5 Dioleate, Polyglyceryl-6 Caprylate, Polyglyceryl-6 Tricaprylate, Polyglyceryl-10 Laurate, Polyglyceryl-10 Myristat, Polyglyceryl-10 Isostearate, Polyglyceryl-10 Diisostearate, Polyglyceryl-10 Oleate und Polyglyceryl-6 Isostearate.

4. Herstellungsverfahren nach Anspruch 3, **dadurch gekennzeichnet, dass** das Tensid Polyglyceryl-6 Isostearate ist.

5. Herstellungsverfahren nach einem der Ansprüche 1 bis 4, **dadurch gekennzeichnet, dass** die kosmetische Zusammensetzung, als Massenanteile, ausgedrückt durch das Verhältnis zur Gesamtmasse der Zusammensetzung, wenigstens enthält:
- zwischen 0,5 % und 5 %, bevorzugt zwischen 1 % und 4 % inversen Latex;
- zwischen 0,1% und 10 %, bevorzugt zwischen 0,5% und 2 % hydrophiles Tensid, ausgewählt aus den Polyclyerolestern, die bei Raumtemperatur flüssig sind und mit HLB höher als oder gleich 8;
- zwischen 70 % und 95 %, bevorzugt zwischen 80 % und 85 % wässrige Phase;
- zwischen 1 % und 20 %, bevorzugt zwischen 3 % und 6 % Fettphase;
- optional zwischen 0,1 % und 0,3 % Parfüm;
- optional zwischen 0,1 % und 1 % Konservierungsstoff.

6. Herstellungsverfahren nach einem der Ansprüche 1 bis 5, **dadurch gekennzeichnet, dass** die Fettphase wenigstens einen Zusatzstoff umfasst, ausgewählt unter den Fettestern, den Pflanzenölen, den Mineralölen, den Silikonen und den öligen Mazeraten.

7. Herstellungsverfahren nach einem der Ansprüche 1 bis 6, **dadurch gekennzeichnet, dass** die Fettphase und/oder die wässrige Phase wenigstens einen Wirkstoff enthalten, der ausgewählt ist aus den feuchtigkeitsspendenden und weichmachenden, alterungsvorbeugenden, beruhigenden, hautfettregulierenden, straffenden, pigmentstörungsausgleichenden und oxidationsverhindernden Wirkstoffen.

8. Herstellungsverfahren nach einem der Ansprüche 1 bis 7, **dadurch gekennzeichnet, dass** die wässrige Phase wenigstens einen Wirkstoff umfasst, der ein pflanzliches Extrakt ist, ausgewählt aus Fucus-Extrakt, Aloe-Vera-Extrakt, Gingko-Biloba-Extrakt, Centella-Asiatica-Extrakt, Wildem-Stiefmütterchen-Extrakt und Calendula-Extrakt.

9. Herstellungsverfahren nach einem der Ansprüche 1 bis 8, **dadurch gekennzeichnet, dass** die Zusammensetzung eine Creme, ein cremeartiges Gel, ein Fluid oder ein Serum ist.

10. Herstellungskit für eine Zusammensetzung, hergestellt nach dem Verfahren nach einem der Ansprüche 1 bis 9, das wenigstens umfasst:
- ein 1. Gefäß, wenigstens die wässrige Phase enthaltend,
- ein 2. Gefäß, wenigstens die Fettphase, den inversen Latex und das hydrophile Tensid enthaltend, das ausgewählt ist aus den Polyclyerolestern, die bei Raumtemperatur flüssig sind und mit HLB höher als oder gleich 8.

## Claims

1. A method for preparing a cosmetic composition in the form of an oil-in-water emulsion, **characterized in that** it comprises at least the following steps:
a) a 1^{st} mixture is prepared, comprising at least one fatty phase, an inverse latex and a hydrophilic surfactant which is selected from the polyglycerol esters that are liquid at ambient temperature and HLB (« HLB » being the acronym of « Hydrophilic-Lipophilic Balance ») greater than or equal to 8;
b) an aqueous phase is added to this 1^{st} mixture so as to obtain a 2^{nd} mixture;
c) optionally, if in the 2^{nd} mixture on completion of step b) no oil-in-water emulsion was formed spontaneously, this 2^{nd} mixture is homogenized until obtaining an oil-in-water emulsion of said cosmetic composition,
the ratio of the mass percentage of said surfactant to the mass percentage of said inverse latex being comprised between 0.1 and 1, more preferably comprised between 0.15 and 0.6, said mass percentages being expressed with respect to the total mass of said cosmetic composition.

2. The preparation method according to claim 1, **characterized in that** all its steps are carried out at ambient temperature.

3. The preparation method according to claim 1 or 2, **characterized in that** the polyglycerol ester is selected from the group constituted by polyglyceryl-2 caprate, polyglyceryl-2 laurate, polyglyceryl-3 laurate, polyglyceryl-4 laurate, polyglyceryl-4 isostearate, polyglyceryl-4 oleate, polyglyceryl-5 laurate, polyglyceryl-5 oleate, polyglyceryl-5 dioleate, polyglyceryl-6 caprylate, polyglyceryl-6 tricaprylate, polyglyceryl-10 laurate, polyglyceryl 10 myristate, polyglyceryl-10 isostearate, polyglyceryl-10 diisostearate, polyglyceryl-10 oleate, and polyglyceryl-6 isostearate.

4. The preparation method according to claim 3, **characterized in that** the surfactant is polyglyceryl-6 isostearate.

5. The preparation method according to any one of claims 1 to 4, **characterized in that** said cosmetic composition comprises, in mass percentages expressed with respect to the total mass of said composition, at least:
- between 0.5% and 5%, preferably between 1% and 4%, of inverse latex;
- between 0.1% and 10%, preferably between 0.5% and 2%, of hydrophilic surfactant selected from the polyglycerol esters that are liquid at ambient temperature and HLB greater than or equal to 8;
- between 70% and 95%, preferably between 80% and 85%, of aqueous phase;
- between 1% and 20%, preferably between 3% and 6%, of fatty phase;
- optionally between 0.1% and 0.3%, of perfume;
- optionally between 0.1% and 1%, of conservative.

6. The preparation method according to any one of claims 1 to 5, **characterized in that** the fatty phase comprises at least one adjuvant selected from fatty esters, vegetable oils, mineral oils, silicones and oily macerates.

7. The preparation method according to any one of claims 1 to 6, **characterized in that** the fatty phase and/or the aqueous phase comprises at least one active ingredient which is selected from the moisturizing and emollient, anti-aging, soothing, sebum regulating, slimming, anti-stain and antioxidant active ingredients.

8. The preparation method according to any one of claims 1 to 7, **characterized in that** the aqueous phase comprises at least one active ingredient which is a plant extract selected from the Fucus extract, the Aloe Vera extract, the Gingko Biloba extract, the Centella Asiatica extract, the wild pansy extract and the Calendula extract.

9. The preparation method according to any one of claims 1 to 8, **characterized in that** said cosmetic composition is a cream, a creamy gel, a fluid or a serum.

10. A kit for preparing a cosmetic composition prepared according to the preparation method according to any one of claims 1 to 9 which comprises at least:
- a 1^{st} container containing at least the aqueous phase,
- a 2^{nd} container containing at least the fatty phase, the inverse latex and the hydrophilic surfactant which is selected from the polyglycerol esters that are liquid at ambient temperature and HLB greater than or equal to 8.
